(19) Europäisches Patentamt
European Patent Office
Office européen des brevets

(11) **EP 4 453 860 B1**

(12) **EUROPEAN PATENT SPECIFICATION**

(45) Date of publication and mention
of the grant of the patent:
**10.12.2025 Bulletin 2025/50**

(21) Application number: **22835076.5**

(22) Date of filing: **16.12.2022**

(51) International Patent Classification (IPC):
**G06T 7/00** (2017.01)

(52) Cooperative Patent Classification (CPC):
**G06T 7/0012;** G06T 2207/10081;
G06T 2207/10084; G06T 2207/10104;
G06T 2207/20084; G06T 2207/30056;
G06T 2207/30081; G06T 2207/30096

(86) International application number:
**PCT/EP2022/086291**

(87) International publication number:
**WO 2023/117740 (29.06.2023 Gazette 2023/26)**

(54) **SYSTEM AND METHOD FOR CLASSIFYING LESIONS**

SYSTEME UND VERFAHREN ZUR KLASSIFIZIERUNG VON LÄSIONEN

SYSTÈME ET PROCÉDÉ DE CLASSIFICATION DE LÉSIONS

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **21.12.2021 IN 202141059703
10.01.2022 EP 22150655**

(43) Date of publication of application:
**30.10.2024 Bulletin 2024/44**

(73) Proprietor: **Koninklijke Philips N.V.
5656 AG Eindhoven (NL)**

(72) Inventors:
 • **MYSORE SIDDU, Dinesh
5656 AG Eindhoven (NL)**
 • **PAWAR, Anil
5656 AG Eindhoven (NL)**

 • **CHAUDHURY, Sudipta
5656 AG Eindhoven (NL)**
 • **HEDDESE SHASTRY, Arun
5656 AG Eindhoven (NL)**

(74) Representative: **Philips Intellectual Property &
Standards
High Tech Campus 52
5656 AG Eindhoven (NL)**

(56) References cited:
WO-A1-2020/190821     WO-A2-2019/136349
US-A1- 2020 245 960

 • **CHADDAD AHMAD ET AL: "Deep Radiomic
Analysis to Predict Gleason Score in Prostate
Cancer", IEEE ACCESS, IEEE, USA, vol. 8, 14
September 2020 (2020-09-14), pages 167767 -
167778, XP011810484, DOI: 10.1109/
ACCESS.2020.3023902**

**Description**

FIELD OF THE INVENTION

The present invention relates to a system, a method and a computer program for classifying lesions, as well as to a method for choosing a set of image features and a machine learning architecture for use by the system or in the method for classifying lesions.

BACKGROUND OF THE INVENTION

Classifying lesions according to known lesion classification schemes can support optimal treatment in clinical practice. With the ever-increasing quality of medical imaging, image-based diagnosis tools have become the standard for many applications. However, lesions are usually still classified based on lesion samples extracted by biopsy. In particular, this is the case for prostate cancer, which is the second most commonly occurring cancer and fifth major cause of death among men worldwide. In fact, unless the lesion region is not easily accessible via biopsy, biopsy-based diagnosing could still be considered the gold standard for most lesion types, including also, for instance, inflammatory disorders, such as in the kidney or the liver, and various kinds of infections. Early diagnoses and treatment planning are generally of high medical significance. In particular, they are crucial in reducing the mortality rate due to prostate cancer. Since both the biopsies needed for diagnoses as well as the treatment of lesions, such as by radiation, for instance, usually rely on image-guidance, an accurate identification of lesion regions, particularly regarding their position and size, in the guiding images by an image-based lesion classification would be desirable. In fact, it would be desirable to eventually facilitate purely image-based diagnoses of lesions in order to avoid any non-necessary invasive steps. There is therefore a need in the art to improve the image-based classification of lesions, particularly prostate tumor regions.

In the prior art, machine learning has been employed in attempts to satisfy this need, wherein different machine learning architectures working with different sets of image features for classifying the lesions have been proposed. However, the known attempts show that there is still room for improving the performance of image-based lesion classification.

US 2020/0245960 A1 describes an automated analysis of three-dimensional medical images of a subject in order to automatically identify specific three-dimensional volumes within the three-dimensional images that correspond to specific anatomical regions. The identification of one or more such volumes can be used to automatically determine quantitative metrics that represent uptake of radiopharmaceuticals in particular organs and/or tissue regions. These uptake metrics can be used to assess a disease state in a subject, determine a prognosis for a subject, and/or determine efficacy of a treatment modality.

SUMMARY OF THE INVENTION

It is an object of the present invention to provide a system, a method and a computer program for classifying lesions with an increased accuracy, as well as a method for choosing a set of image features and a machine learning architecture for use by the system or in the method for classifying lesions such that the lesion classification accuracy is increased.

In a first aspect, the present invention relates to a system for classifying lesions, wherein the system comprises an image providing unit for providing a dual-modal image of an imaging region, the dual-modal image comprising a computed tomography (CT) image and a positron emission tomography (PET) image registered to each other, wherein the imaging region includes a) a tissue region of interest comprising a lesion to be classified and b) a reference tissue region comprising healthy tissue. The system further comprises an identifying unit for identifying, in each of the CT image and the PET image, a respective lesion image segment corresponding to the lesion in the tissue region of interest and, in the PET image, a reference image segment corresponding to the reference tissue region. Moreover, the system comprises a normalizing unit for normalizing the lesion image segment in the PET image with respect to the reference image segment in the PET image, an image feature extracting unit for extracting values for a set of image features associated with the lesion from the lesion image segment in the CT image and from the normalized lesion image segment in the PET image, and a classifying unit for classifying the lesion according to its severity based on the extracted image feature values.

Since a dual-modal image comprising a CT and a PET image is used, wherein the lesion is classified based on values of image features extracted from both the CT and the PET image, the benefits of anatomical and functional imaging are combined, thereby allowing for an accurate lesion classification. Since physiological variations as well as variations in the PET imaging modality and the parameters chosen for it, which would otherwise affect the PET image and therefore also the image feature values extracted from the lesion image segment in the PET image, are accounted for by normalizing the lesion image segment in the PET image with respect to a reference image segment, the lesion classification accuracy can be further increased.

The image providing unit is adapted to provide a dual-modal image of an imaging region, wherein the dual-modal image comprises, particularly may consist of, a CT image and a PET image registered to each other. The CT image and the PET image can be acquired using an integrated imaging system comprising a CT imaging modality and a PET imaging modality. They are preferably volumetric, i.e. three-dimensional images, but can also be two-dimensional images. In case they are volumetric images, they may be decomposed in terms of two-dimensional image slices along a given axis, such as a scanning axis, for instance. The image elements of the CT image may be associated with image values given in Hounsfield units (HU), and the image elements of the PET image may be associated with image values given in standardized uptake values (SUVs).

The registration between the CT image and the PET image may be achieved according to any known registration technique. By virtue of the registration between the CT image and the PET image, which both show the imaging region, the dual-modal image can be thought of as associating, to each point in the imaging region, two image elements, namely one image element in the CT image and one image element in the PET image.

The imaging region includes a tissue region of interest that comprises a lesion to be classified and, furthermore, a reference tissue region comprising healthy tissue. The tissue region of interest may be a part of an organ or be a whole organ containing the lesion that is to be classified, for instance. The reference tissue region may refer to a part of an organ or be a whole organ that comprises, at least supposedly and/or substantially, healthy tissue, i.e., for instance, as compared or in contrast to the tissue region of interest. Hence, the tissue comprised by the reference tissue region being "healthy" may refer to the tissue being healthy as compared to the tissue region of interest, for instance. The reference tissue region may preferably be chosen such that it behaves similarly to, or even like, the tissue region of interest in response to physiological variations and/or variations in the PET imaging modality and the parameters chosen for it. The imaging region may, for instance, comprise or consist of the body or a part of the body, such as the upper body, of a patient.

The identifying unit is adapted to identify, in each of the CT image and the PET image, a respective lesion image segment corresponding to the lesion in the tissue region of interest and, in the PET image, a reference image segment corresponding to the reference tissue region. Hence, the identifying unit is adapted to identify lesion image segments in both the CT and the PET image and a, possibly single, reference image segment in the PET image. By virtue of the registration between the CT and the PET image, the identifying unit may also be viewed as identifying a joint lesion image segment, i.e. a segment in the dual-modal image corresponding to the lesion in the tissue region of interest, wherein the joint lesion image segment may be understood as a combination of the lesion image segments in the CT and the PET image, or an overlay image segment corresponding thereto. In fact, by virtue of the registration between the CT and the PET image, the identified reference image segment in the PET image also corresponds to a reference image segment in the CT image corresponding to the reference tissue region.

In an embodiment, the identifying unit can be adapted to identify, in each of the CT image and the PET image, a respective reference image segment corresponding to the reference tissue region, wherein the reference image segment in the PET image is identified as the image segment registered to the reference image segment identified in the CT image. This allows for a particularly precise identification of the reference image segment in the PET image.

The identifying unit can be adapted to identify the respective lesion image segments in the CT image and the PET image as well as the reference image segment in the PET image, and possibly also the reference image segment in the CT image, based on a user input or based on an automatic segmentation process. Preferably, at least the reference image segment in the PET image, and possibly also in the CT image, is identified based on an automatic segmentation process.

The normalizing unit is adapted to normalize the lesion image segment in the PET image with respect to the reference image segment in the PET image. Normalizing the lesion image segment refers to a normalization of the image values in the lesion image segment. For instance, the normalizing unit can be adapted to normalize the SUVs in the lesion image segment of the PET image with respect to a median of the SUVs in the reference image segment of the PET image. In fact, the normalizing unit might be adapted to normalize the whole PET image with respect to the reference image segment in the PET image by, for instance, normalizing the SUVs of the whole PET image with respect to a median of the SUVs in the reference image segment of the PET image.

While for CT imaging a standardized scale in terms of Hounsfield units exists, for PET imaging no such standardized scale of image values is readily available. The detected intensities based on which image values are derived in PET imaging highly depend on the amount of nuclear tracer injected, the scintillation material used in the detector, whether time of flight is accounted for or not, and the reconstruction parameters used in the PET imaging modality. Usually, therefore, close attention to details is required in PET scans to avoid errors. Normalizing the lesion image segment in the PET image with respect to a reference image segment in the PET image allows for a lesion classification that is less sensitive against such manual and systematic errors.

The image feature extracting unit is adapted to extract values of a set of image features associated with the lesion from the lesion image segment in the CT image and from the normalized lesion image segment in the PET image. Hence, the set of image features includes CT image features and PET image features. Preferably, the set of image features includes radiomics features associated with the lesion, particularly semantic features like a size, a shape and/or a location of the lesion, and/or agnostic features like texture-based first order statistics associated with the lesion, and/or features resulting from higher-order algorithms like gray-level co-occurrence matrices (GLCM) and/or a gray-level run length matrix (GLRLM). The first-order features are based on statistical information of the image and can include a maximum intensity, a minimum intensity, an average intensity, a standard deviation of the intensity, a variance of the intensity, an energy, an entropy, a sharpness, a skewness, a kurtosis and/or a gray-scale. These features can give statistical information based on the frequency distribution of different gray-levels in the image. Lesion classification based on extracted radiomics features may be viewed as involving radiomics feature analysis, which generally may be defined as the extraction of quantitative image features for the characterization of disease patterns.

In an example, the lesion image segments in the CT and the PET image may be cuboid-shaped image regions showing the lesion and its immediate surrounding in the tissue region of interest, wherein the respective cuboid-shaped image region, or its margin, might also be referred to as a region of interest bounding box. The radiomics features may then be extracted from the respective image values, or intensities, in these rectangular image regions.

The classifying unit is adapted to classify the lesion according to its severity based on the extracted image feature values. For instance, the classification of the lesion may be achieved based on a, possibly predefined, relation between values of the extracted image features and known severity grades. A predefined relation may be stored by or for access by the classifying unit in the form of a table, for instance. Particularly if the number of image features becomes large, the table might also be viewed as a database relating values of image features associated with lesions to assigned severity classes, wherein the assignment might be based on an assessment of lesion severity by trained pathologists. Preferably, however, the classifying unit is adapted to classify the lesions based on a predetermined procedure, possibly stored by or for access by the classification unit in terms of a computer program. The predetermined procedure might have the form of an artificial intelligence, particularly a machine learning model, that has undergone supervised learning.

Since the lesion is classified according to its severity, an optimal biopsy target and/or an optimal treatment can be determined for the lesion depending on the classification. For instance, if the lesion is a tumor, its class may indicate whether it is malignant or benign, wherein a malignant tumor might require intensive treatment while a benign tumor might require less or no treatment at all. Moreover, accurately locating malignant tumors also allows for an optimal biopsy targeting and/or an optimal planning of a subsequent ablation treatment, for instance. The use of image features of both the CT and the PET image allows for an increased accuracy in classifying the lesions, and therefore also for an improved targeting of biopsies for diagnosis and/or an improved treatment targeting.

In a preferred embodiment, the tissue region of interest refers to a patient's prostate, the lesion is a prostate tumor region and the PET image is generated using an imaging substance binding to prostate-specific membrane antigen (PSMA). In particular, the lesion to be classified can be a single habitat, or set of contiguous habitats, of a prostate tumor, and the imaging substance, which might also be referred to as a PET tracer, may particularly comprise gallium-68 ($^{68}$Ga), in which case the imaging technique used may be referred to as $^{68}$Ga-PSMA-PET/CT imaging. More specifically, the imaging substance can be $^{68}$Ga-PSMA-11. Over 90% of prostate cancers overexpress PSMA. Hence, these tumor cells may be accurately tagged for diagnosis by using a PET imaging substance binding to PSMA. The molecular imaging technique which is referred to as $^{68}$Ga-PSMA-PET/CT imaging appears clinically to have superseded CT imaging alone, and appears superior to magnetic resonance (MR) imaging, for the detection of metastatic diseases. $^{68}$Ga-PSMA-PET/CT imaging has the ability to identify the stage of prostate cancer with high reliability at presentation and can help informing an optimal treatment approach.

In a preferred embodiment, the lesion is classified according to a binary classification of severity based on a Gleason score, the binary classification distinguishing normal/indolent from aggressive prostate tumor regions. Gleason grading is the most commonly used prostate cancer prognosis tool by doctors. It has been used for a long time to determine the aggressiveness of prostate cancer for planning treatment options. However, usually the process requires well-trained pathologists to look at multiple biopsy samples under the microscope and assign Gleason grades to the cancer based on its severity as assessed by the pathologists. The system disclosed herein allows for a purely image-based, therefore non-invasive, and objective determination of Gleason grades of prostate cancer.

In prostate tumors, different habitats with distinct volumes are present, each with a specific combination of flow, cell density, necrosis and edema. Habitat distribution in patients with prostate cancer can be used to discriminate between cancer regions that progress quickly and those that are more indolent and, in particular, in order to classify the cancer regions according to the Gleason grading scheme based on $^{68}$Ga-PSMA-PET/CT images with high accuracy. A deep understanding of tumor heterogeneity can be obtained by a regional analysis in which image elements, such as voxels, for instance, corresponding to a tumor are assigned a specific color depending on the combination of intensity, and the clusters of voxels with specific colors yield regions that reflect different habitats, which might be referred to as physiological microenvironments, linking it with a Gleason score and with the hormonal status, as well as with the neuroendocrine component within the tumor.

In a preferred embodiment, the reference tissue region is a patient's liver, particularly the liver of the patient whose prostate the tissue region of interest refers to. Hence, the identifying unit can be adapted to identify, in the PET image, a reference image segment corresponding to the patient's liver. The liver contains the largest amount of blood in the body at any given time. Hence, the contrast in PET image values, such as SUVs, between the liver and the tissue region of interest including the lesion to be classified is directly correlated with the patient's circulatory system. Using the liver as the reference tissue region therefore allows for a normalization of the lesion image segment that eliminates the variability of patient physiology particularly well. However, in principle, also other organs, preferably normal or healthy organs, might be chosen as reference tissue regions. Choosing the liver as reference tissue region might be preferred specifically in case the lesion to be classified is a prostate tumor region.

In a preferred embodiment, the set of image features includes, as features whose values are to be extracted from the lesion image segment in the CT image, the total energy, a maximum two-dimensional diameter in row direction, a sphericity, a surface area, a large area emphasis and a dependence entropy, and, as features whose values are to be extracted from the normalized lesion image segment in the PET image, a $90^{th}$ percentile, a minimum, a flatness and a sphericity. Classifying lesions based on values of this combination of image features allows for particularly accurate results. Optionally, the set of image features can include or consist of any combination of one or more of the mentioned features whose values are to be extracted from the lesion image segment in the CT image (i.e., the total energy, a maximum two-dimensional diameter in row direction, a sphericity, a surface area, a large area emphasis and a dependence entropy) and one or more of the mentioned features whose values are to be extracted from the normalized lesion image segment in the PET image (i.e., a $90^{th}$ percentile, a minimum, a flatness and a sphericity). This may still allow for very accurate classification results.

In a preferred embodiment, the identifying unit comprises, for identifying the reference image segment in the CT image, a machine learning architecture adapted, and preferably trained, for receiving a CT image of the imaging region including the reference tissue region as an input and determining the reference image segment in the CT image as an output. The reference image segment in the PET image can then be identified as the image segment of the PET image corresponding to the determined reference image segment in the CT image via the registration between the CT and the PET image.

In particular, if the CT image is a volumetric image, the machine learning architecture may be adapted to receive the CT image in slices, wherein each input is formed by three adjacent image slices. For instance, if the CT image contains 100 slices, three contiguous slices may be provided at a time as an input to the machine learning architecture. The machine learning architecture may be trained using binary cross-entropy and a loss function.

The identifying unit can be adapted to identify the reference image segment in the CT image by first modifying the CT image and then identifying the reference image segment in the modified CT image. The rescaling may refer to a clipping of CT image values to a range, or window, including image values corresponding to the reference tissue region and image values corresponding to the tissue region of interest, and a subsequent mapping of the range, or window, to a full range of possible CT image values, i.e., for instance, intensities. For instance, the CT image data may be clipped to a range between -150 and 250 HU, wherein this range is subsequently mapped to the range from 0 to 255 HU. Liver and lesion soft-tissues typically lie in the range between -150 and 250 HU.

In a preferred embodiment, the machine learning architecture comprised by the identifying unit comprises a convolutional neural network with convolutional, activation and pooling layers as a base network and, at a plurality of convolutional stages of the base network, a respective side set of convolutional layers connected to a respective end of the convolutional layers of the base network at the respective convolutional stage, wherein the reference image segment in the CT image is determined based on outputs of the plurality of side sets of convolutional layers. In particular, the base network can correspond to a VGG-16 architecture with the last fully connected layers removed as described in the conference contributions "Detection-aided liver lesion segmentation using deep learning" by M. Bellver et al., Conference of Neural Information Processing, pages 1 to 4 (2017), arXiv:1711.11069, and "Deep Retinal Image Understanding" by K.-K. Maninis et al., Medical Image Computing and Computer-Assisted Intervention, vol. 9901, pages 140 to 148 (2016), arXiv:1609.01103. However, the base network may also comprise, for instance, a U-Net or Unet++ architecture.

The machine learning architecture comprised by the identifying unit can be trained, for instance, on ImageNet, which allows for a fast and robust model training. More specifically, the machine learning architecture comprised by the identifying unit can be trained on a set of training CT and/or PET images showing reference tissue regions and/or lesions in tissue regions of interest of the type to be identified, wherein the training images might be or become a subset of the images on ImageNet. In each training image, a lesion image segment and/or a reference image segment corresponding to the reference tissue region and/or the lesion in the tissue region of interest may be identified in preparation of training, i.e. by a pathologist, for instance, wherein this identification in preparation of training, which can refer to an annotation, indication and/or a segmentation, for instance, might be considered as a ground truth for training. In the case of the reference tissue region being a patient's liver, the segmentation process automated by the trained machine learning architecture of the identifying unit can give results corresponding to a dice score of 0.96, for instance. Via training, a chosen machine learning architecture, which might initially implement a general machine learning model, is tuned to become suitable for identifying reference image segments and/or lesion image segments, wherein the tuning can involve, in particularly, tuning hidden parameters of the architecture. According to a more general training scheme, also other parameters, such as hyperparameters, of the architecture can be tuned. Hyperparameters may be viewed as parameters of a given type of machine learning architecture, which control its learning process.

In a preferred embodiment, the classifying unit comprises, for classifying the lesion, a machine learning architecture suitable, and possibly already trained, for receiving image feature values as an input and determining an associated lesion severity class as an output. Hence, the system may comprise two separate machine learning architectures, i.e. a first machine learning architecture of the identifying unit for identifying the reference tissue region in the CT and/or the PET image, wherein the first machine learning architecture might be referred to as an identifying machine learning architecture, and a second machine learning architecture of the classifying unit for classifying a lesion based on image feature values extracted from lesion image segments in the CT and the PET image, wherein the second machine learning architecture might be referred to as a classifying machine learning architecture.

The machine learning architecture comprised by the classifying unit can be trained using the same training data as for the machine learning architecture comprised by the identifying unit, i.e., for instance, on ImageNet, which allows for a fast and robust model training. More specifically, the machine learning architecture comprised by the classifying unit can be trained based on a set of training CT and/or PET images showing lesions in tissue regions of interest of the type to be classified, which might be or become a subset of the images on ImageNet, or based on lesion image segments identified therein. From each training image or lesion image segment used for training, respective image feature values associated with the respective lesion therein might be extracted, wherein to the respective training image, lesion image segment or extracted image feature values, a class, such as a lesion severity class, may be assigned in preparation of training, i.e. by a pathologist, for instance, wherein this assignment in preparation of training might be considered as a ground truth for training. Via training, a chosen machine learning architecture, which might initially implement a general machine learning model, is tuned to become suitable for classifying lesions, wherein the tuning can particularly involve tuning hidden parameters of the architecture. According to a more general training scheme, also other parameters, such as hyperparameters, of the architecture can be tuned.

For instance, the machine learning architecture comprised by the classifying unit, particularly the trained one, can be a random forest with entropy as a splitting criterion, the maximum tree depth being 5, the maximum number of features considered for each split being 2 and the maximum number of trees being 500. However, different hyperparameters for the random forest may prove to be preferred depending on the training dataset used for training the machine learning architecture.

In some embodiments, the set of image features and the classifying machine learning architecture are predetermined by training candidate machine learning architectures for classifying lesions using values of candidate sets of image features extracted from training images of tissue regions comprising lesions with assigned respective lesion severity classes, determining performances of the respective trained candidate machine learning architectures for values of the respective candidate sets of image features extracted from test images of tissue regions comprising lesions with assigned respective lesion severity classes, and choosing the candidate set of image features and the candidate machine learning architecture to be used in the system based on the determined performances. Hence, the machine learning architecture of the classifying unit and the set of image features used for classification may be tuned, i.e. determined, in a joint training process. This allows for an efficient and accurate classification of lesions by the system, since the machine learning architecture of the classifying unit and the set of image features used for classification are adapted to each other.

In a further aspect of the invention, a method for classifying lesions is presented, wherein the method comprises a step of providing a dual-modal image of an imaging region, the dual-modal image comprising a CT image and a PET image registered to each other, wherein the imaging region includes a) a tissue region of interest comprising a lesion to be classified and b) a reference tissue region comprising healthy tissue, and a step of identifying, in each of the CT image and the PET image, a respective lesion image segment corresponding to the lesion in the tissue region of interest and, in the PET image, a reference image segment corresponding to the reference tissue region. The method further comprises the steps of normalizing the lesion image segment in the PET image with respect to the reference image segment in the PET image, extracting values of a set of image features associated with the lesion from the lesion image segment in the CT image and from the normalized lesion image segment in the PET image, and classifying the lesion according to its severity based on the extracted image feature values. The method may, for instance, be executed using a system as described above, particularly using an identifying machine learning architecture and/or, in particular, a classifying machine learning architecture as described above.

In another aspect, a method for choosing a set of image features and a machine learning architecture, particularly the classifying machine learning architecture, for use by the system or in the method for classifying lesions is presented, which is subsequently also referred to as a tuning and training method, wherein the method comprises the steps of i) providing a set of dual-modal training images, each training image comprising a CT image and a PET image registered to each other, in each of which a respective lesion image segment corresponding to a respective lesion in a tissue region of interest has been identified, wherein, for each of the lesions in the training images, an assigned severity class is provided, and ii) choosing a candidate set of image features from a predefined collection of possible image features. The method further comprises iii) generating a training dataset by extracting, from each of the lesion image segments identified in the training images, corresponding values of each of the candidate set of image features and assigning them to the respective severity class assigned to the lesion in the respective training image, iv) choosing a candidate machine learning architecture suitable for receiving image feature values as an input and determining an associated lesion severity class as an output, and v) training the candidate machine learning architecture on the training dataset. Moreover, the method comprises the steps of vi) providing a set of dual-modal test images, each test image comprising a CT and a PET image, in each of which a respective lesion image segment corresponding to a respective lesion in a tissue region of interest has been identified, wherein, for each of the lesions in the test images, an assigned severity class is provided, and vii) generating a test dataset by extracting, from each of the lesion image segments identified in the test images, corresponding values of each of the candidate set of image features and assigning them to the respective severity class assigned to the lesion in the respective test image. The method further comprises viii) determining a performance of the candidate machine learning architecture on the test dataset based on a relation between the assigned severity classes provided for the lesions in the test images and the corresponding lesion severity classes determined by the candidate machine learning architecture, ix) repeating the above steps, i.e. steps i) to xiii), with increasing numbers of image features in the candidate set of image features, wherein for each repetition, the candidate set of image features is enlarged by a further image feature from the predefined collection of possible image features, the further image feature being the one that results in the highest increase in performance, until an abort criterion is met, and x) repeating the above steps, i.e. steps i) to ix), with at least one, but preferably a plurality of candidate machine learning architectures, wherein the set of image features and the machine learning architecture to be used for classifying lesions are chosen from the respective candidates according to the determined performances.

The relation between the assigned severity classes provided for the lesions in the test images and the corresponding lesion severity classes determined by the candidate machine learning architecture, based on which the performance is determined, may refer to a one-by-one comparison, for instance. More generally, the performance may be a measure of how often the severity class determined by the candidate machine learning model coincides, possibly at least up to some error margin, with the severity class assigned to the respective lesion image segment in the test images. Thus, for instance, the performance may refer to a classification accuracy.

In some embodiments, the values of the image features extracted from the lesion image segments identified in the training images and the test images, respectively, are Gaussian standardized, i.e. rescaled based on their mean and their standard deviation such that they form a standard normal distribution. This avoids attributing a wrong significance to features with higher magnitude in their value. The mean and standard deviation used for rescaling the value of a particular feature extracted from a lesion image segment in a given image can, for instance, correspond to the mean and, respectively, standard deviation of the values of the particular feature previously extracted from lesion image segments in other images, particularly training images.

In a preferred embodiment, the plurality of candidate machine learning architectures correspond to the same type of machine learning architecture, particularly random forests, but with different hyperparameters. The hyperparameters are preferably chosen successively according to a grid search. The test images may include different subsets of the training images, thereby performing cross validation. However, the test images may also be separate images not included in the whole set of training images.

In another aspect, a system for carrying out the method for choosing a set of image features and a machine learning architecture, particularly the classifying machine learning architecture, for use by the system or in the method for classifying lesions is presented, which is subsequently also referred to as a tuning and training system. The system comprises, for each of the steps i) to x), units for carrying out the respective one of the steps i) to x). That is to say, the system comprises a providing unit for i) providing a set of dual-modal training images, each training image comprising a CT image and a PET image registered to each other, in each of which a respective lesion image segment corresponding to a respective lesion in a tissue region of interest has been identified, wherein, for each of the lesions in the training images, an assigned severity class is provided, and a choosing unit for ii) choosing a candidate set of image features from a predefined collection of possible image features. The system further comprises a generating unit for iii) generating a training dataset by extracting, from each of the lesion image segments identified in the training images, corresponding values of each of the candidate set of image features and assigning them to the respective severity class assigned to the lesion in the respective training image, a choosing unit for iv) choosing a candidate machine learning architecture suitable for receiving image feature values as an input and determining an associated lesion severity class as an output, and a training unit for v) training the candidate machine learning architecture on the training dataset. Moreover, the system comprises a providing unit for vi) providing a set of dual-modal test images, each test image comprising a CT and a PET image, in each of which a respective lesion image segment corresponding to a respective lesion in a tissue region of interest has been identified, wherein, for each of the lesions in the test images, an assigned severity class is provided, and a generating unit for vii) generating a test dataset by extracting, from each of the lesion image segments identified in the test images, corresponding values of each of the candidate set of image features and assigning them to the respective severity class assigned to the lesion in the respective test image. The system further comprises a determining unit for viii) determining a performance of the candidate machine learning architecture on the test dataset based on a relation between the assigned severity classes provided for the lesions in the test images and the corresponding lesion severity classes determined by the candidate machine learning architecture, wherein the units of the system are adapted to ix) repeat the respective steps i) to xiii) with increasing numbers of image features in the candidate set of image features, wherein for each repetition, the candidate set of image features is enlarged by a further image feature from the predefined collection of possible image features, the further image feature being the one that results in the highest increase in performance, until an abort criterion is met, and to x) repeat the respective steps i) to ix) with at least one, but preferably a plurality of candidate machine learning architectures, wherein the set of image features and the machine learning architecture to be used for classifying lesions are chosen from the respective candidates according to the determined performances.

In yet a further aspect, a computer program for classifying lesions is presented, wherein the program comprises program code means for causing the system for classifying lesions to execute the method for classifying lesions when the program is run on a computer controlling the system. Also, a computer program for choosing a set of image features and a machine learning architecture, particularly the classifying machine learning architecture, for use by the tuning and training system or in the tuning and training method is presented, wherein the program comprises program code means for causing the tuning and training system to execute the tuning and training method when the program is run on a computer controlling the tuning and training system. In this aspect, the respective system may actually be the computer itself, wherein the respective computer program may be executed by one or more processing units of the computer.

It shall be understood that the system of claim 1, the method of claim 10, the method of claim 11 and the computer program of claim 14 have similar and/or identical preferred embodiments, in particular as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

## BRIEF DESCRIPTION OF THE DRAWINGS

In the following drawings:

Fig. 1 shows schematically and exemplarily the system for classifying lesions,
Fig. 2 shows schematically and exemplarily a base network underlying a machine learning architecture comprised by the identifying unit,
Fig. 3 shows schematically and exemplarily the method for classifying lesions,
Fig. 4 illustrates schematically and exemplarily steps taken in an embodiment to normalize a lesion image segment in a PET image,
Fig. 5 illustrates schematically and exemplarily steps taken in an embodiment of the method for choosing a set of image features and a machine learning architecture for use by the system or in the method for classifying lesions,
Fig. 6 illustrates schematically and exemplarily embodiments according to several of the aspects described above,
Fig. 7A illustrates the classification accuracy of the system for cross-validation in an embodiment in terms of a confusion matrix,
Fig. 7B illustrates the classification accuracy of the system for test data in an embodiment in terms of a confusion matrix,
Fig. 8 illustrates the classification accuracy of the system in an embodiment in terms of a receiver operator characteristic (ROC), and
Fig. 9 illustrates the dependence of the classification accuracy of the system on the extracted image features in an embodiment.

## DETAILED DESCRIPTION OF EMBODIMENTS

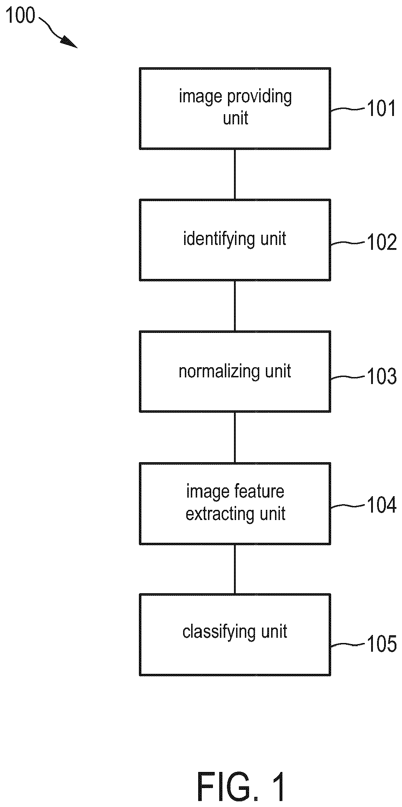

Fig. 1 shows schematically and exemplarily a system 100 for classifying lesions. The system 100 comprises an image providing unit 101 for providing a dual-modal image of an imaging region, the dual-modal image comprising a CT image and a PET image registered to each other, wherein the imaging region includes a) a tissue region of interest comprising a lesion to be classified and b) a reference tissue region comprising healthy tissue.

The CT image and the PET image may be acquired using an integrated imaging system comprising a CT imaging modality, such as a CT scanner, and a PET imaging modality, such as a PET scanner. However, the CT image and the PET image may also have been acquired separately by separate imaging modalities. In case of an integrated imaging system, the registration between the CT and the PET image may be provided by the imaging system. However, it is generally also possible that the registration between the CT image and the PET image is provided separately, particularly after imaging.

The image providing unit may be, for instance, a storage unit storing the dual-modal image for access by other units of the system 100, or it may be an interface via which the system 100 receives the dual-modal image. The image providing unit 101 may also comprise an interface via which the system 100 receives the separate CT and PET images and, furthermore, a processing unit providing a registration between the CT and the PET image, wherein the image providing unit 101 may then be adapted to provide the dual-modal image based on the separate CT and PET images and the registration. The dual-modal image may be viewed as a single image corresponding to an overlay of the CT image and the PET image, wherein the overlay is built based on the registration between the two images.

The system 100 further comprises an identifying unit 102 for identifying, in each of the CT image and the PET image, a respective lesion image segment corresponding to the lesion in the tissue region of interest and, in the PET image, a reference image segment corresponding to the reference tissue region. If the system 100 is adapted to classify prostate tumor regions, such as different habitats within prostate tumors, the lesion image segments may refer to image segments containing the respective prostate tumor region. The lesion image segments may have a standard form, which might be cuboid-shaped, in which case the lesion image segment or its borders might be referred to as a region of interest bounding box. The lesion image segment may then show, apart from the particular prostate tumor region of interest, also surrounding tissue of the prostate and/or prostate tumor.

The lesion image segments may be generated by applying a mask to the respective images, wherein the mask can be generated in response to a user input and/or by the identifying unit alone, such as by employing an identifying machine learning architecture, for instance. In the embodiments described herein, prostate tumor habitats have been marked by trained users of the system, but the lesion image segments may also be identified using deep neural network based segmentation architectures like U-Net or using a dynamic threshold on the PET scan. Identifying the reference image segment in the CT image might, likewise, refer to applying a mask to the CT image.

In case of the lesions to be classified being habitats of prostate tumors and the PET image being generated using an imaging substance binding to PSMA, it may be preferred to identify the lesion image segments, i.e., for instance, to generate bounding boxes around the respective habitats, based on the PET image and transfer the bounding boxes to the CT image via the registration between the PET and the CT image. In turn, it might generally be preferred that the identifying unit is adapted to identify, in each of the CT image and the PET image, a respective reference image segment corresponding to the reference tissue region, wherein the reference image segment in the PET image is identified as the image segment registered to the reference image segment identified in the CT image. Hence, in case of classifying prostate tumor habitats, it might be preferred to identify the lesion image segment in the PET image and the reference image segment in the CT image, wherein the lesion image segment in the CT image and the reference image segment in the PET image are identified via the registration.

For extending the masks in the CT and the PET image to the respective other image via the registration, the resolution of the CT and the PET image may need to be equalized. For instance, CT volumes are usually of a $512 \times 512$ spatial resolution, whereas PET images typically have a spatial resolution of $192 \times 192$. But, for instance, the CT reference image segment mask may be transferred to the PET image by increasing the spatial resolution of the PET image to $512 \times 512$ by trilinear interpolation.

The system 100 further comprises a normalizing unit 103 for normalizing the lesion image segment in the PET image with respect to the reference image segment in the PET image. For instance, the reference tissue region may be a patient's liver, wherein the identifying unit may be adapted to identify, as the reference image segment, an image segment corresponding to the patient's liver, wherein then the normalizing unit may be adapted to normalize the lesion image segment in the PET image, i.e., for instance, the image segment of the PET image corresponding to the prostate tumor habitat to be classified, with respect to the image segment in the PET image corresponding to the patient's liver. The normalization may refer to a normalization of image values in the lesion image segment of the PET image with respect to a characteristic value of the image values in the PET image corresponding to the patient's liver, wherein the characteristic value may be, for instance, a median SUV.

The system 100 further comprises an image feature extracting unit 104 for extracting values of a set of image features associated with the lesion from the lesion image segment in the CT image and from the normalized lesion image segment in the PET image. In particular, the set of image features to be extracted by the image feature extracting unit 104 may be radiomics features, wherein the radiomics features may be extracted from bounding boxes around the lesion to be classified, the bounding boxes being identified in the CT and the PET image, respectively. The set of image features to be extracted by the image feature extracting unit may be a predefined subset from a ground set, i.e. a basic collection, of possible image features, wherein the predefinition may be the result of a training of the system 100 or result from a preconfiguration of the system 100 based on a user input.

The system 100 further comprises a classifying unit 105 for classifying the lesion according to its severity based on the extracted image feature values. Since the lesion is classified based on values of image features extracted from both the CT and the PET image, anatomical and functional information enters the classification. Moreover, since the lesion image segment in the PET image from which the image feature values are extracted is previously normalized with respect to the reference image segment corresponding to the reference tissue region, the functional information can be standardized, thus allowing for an objective and accurate classification of the lesion.

In case the tissue region of interest refers to a patient's prostate and the lesion is a prostate tumor region, wherein the PET image may be generated using an imaging substance binding to PSMA, it may be preferred that the lesion is classified according to a binary classification of severity based on a Gleason score, wherein the binary classification distinguishes indolent from aggressive prostate tumor regions.

In a preferred embodiment, the set of image features to be extracted by the image feature extracting unit 104 includes, particularly consists of, the features listed in following Table 1, in which the left column lists the features whose values are to be extracted from the lesion image segment in the CT image and in which the right column lists the features whose values are to be extracted from the normalized image segment in the PET image.

Table 1: Set of extracted features in an embodiment

| CT features | PET features |
|---|---|
| total energy | 90th percentile |
| maximum 2D diameter in row-direction | minimum |
| sphericity | flatness |
| surface area | sphericity |
| large area emphasis | |
| dependence entropy | |

The features listed in above Table 1 have been found to be the ten most important features for classifying lesions, particularly prostate tumor habitats. In fact, any subset of these features has been found to result in lower accuracies for classifying prostate tumor habitats into indolent and aggressive ones. In an embodiment, therefore, also any combination of one or more of the CT features and one or more of the PET listed in Table 1 could be used. Using more features, particularly using more than the ten features listed in Table 1, on the other hand, might result in a higher computational effort without an adequate increase in classification accuracy.

The importance of using features from both the CT image and the PET image together can be understood from the results of three independent experiments that were performed. In a first of these experiments, only features from the CT image were used. In a second of these experiments, only features from the PET image were used. And in a third of these experiments, concatenated (fused) features from both the CT and the PET image were used. The best results were achieved with the concatenated features, as can be seen from Table 2 below.

Table 2: Performance measured in terms of the area under the receiver-operator characteristic curve (ROC-AUC) for validation on training data (cross-validation) and test data.

| Metric | Only CT features | Only PET features | Fused features |
|---|---|---|---|
| ROC-AUC for cross-validation on training data (%) | 90 | 92 | 94 |
| ROC-AUC for test data (%) | 83 | 85 | 90 |

The identifying unit 102 preferably comprises, for identifying the reference image segment in the CT image, a machine learning architecture adapted and trained for receiving a CT image of an imaging region including a reference tissue region as an input and determining a segment in the CT image corresponding to the reference tissue region as an output. Thus, the machine learning architecture comprised by the identifying unit 102 may be adapted and trained to segment the reference tissue region in a CT image. Its input may consist of a CT image and its output may consist of the CT image with the reference tissue region being segmented in it, or in a correspondingly masked CT image.

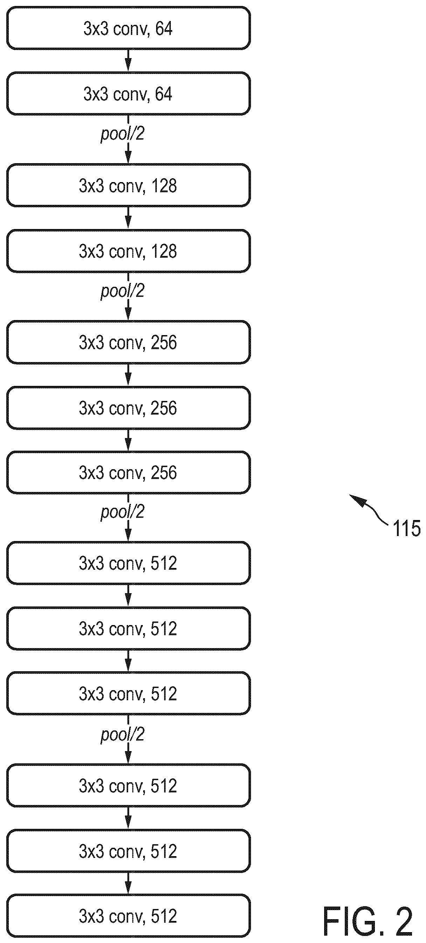

As shown schematically and exemplarily in Fig. 2, the machine learning architecture comprised by the identifying unit 102 may comprise a convolutional neural network with convolutional, activation and pooling layers as a base network 115 and, at a plurality of convolutional stages of the base network 115, a respective side set of convolutional layers connected to a respective end of the convolutional layers of the base network 115 at the respective convolutional stage, wherein the reference image segment in the CT image is determined based on outputs of the plurality of side sets of convolutional layers. The base network 115 may, as seen in Fig. 2, correspond to configuration B of the general VGG-16 architecture originally disclosed in the conference contribution "Very Deep Convolutional Networks for Large-Scale Image Recognition" by K. Simonyan and A. Zisserman, 3rd International Conference on Learning Representations (2015). The filters used for the convolutional layers have a relatively small size of $3 \times 3$, wherein the convolutional layers have depths ranging from 64 to 512. The pooling layers are, in this embodiment, max-pooling layers, wherein, as indicated in Fig. 2 by the shorthand "/2", the max-pooling is performed over a window of size $2 \times 2$.

While Fig. 2 shows only a base network 115, details on the side sets of convolutional layers connected to the ends of the convolutional layers of the base network 115 at the respective convolutional stages, and on how the reference image segment can be determined in the CT image based on outputs of the side sets of convolutional layers, can be inferred from the conference contributions "Detection-aided liver lesion segmentation using deep learning" by M. Bellver et al., Conference of Neural Information Processing, pages 1 to 4 (2017), arXiv:1711.11069, and "Deep Retinal Image Understanding" by K.-K. Maninis et al., Medical Image Computing and Computer-Assisted Intervention, vol. 9901, pages 140 to 148 (2016), arXiv:1609.01103. The activation layers used in the machine learning architecture of the identifying unit may, in particular, be rectified linear unit (ReLU) activation layers.

The base network 115 may be pre-trained on ImageNet. As the network goes deeper, the information it carries becomes coarser and the learned features become more related to semantics. On the other hand, at the shallower feature maps that work at a higher resolution, filters capture more local information. To take advantage of the information learned at feature maps that work at different resolutions, the several side outputs are used in the machine learning architecture comprised by the identifying unit 102. In particular, the side sets of convolutional layers connected to an end of the convolutional layers of the base network 115 at the respective convolutional stage may be provided with supervision, wherein the reference image segment in the CT image is determined based on outputs of the plurality of side sets of convolutional layers provided with supervision. Each of the side outputs, i.e., the sets of convolutional layers connected to the respective ends of the plurality of convolutional stages, specializes on different types of features, depending on the resolution at the respective connection point. In an embodiment, the outputs of the plurality of side sets of convolutional layers may be resized and linearly combined to identify the reference image segment in the CT image, i.e., for instance, to generate a segmented liver mask.

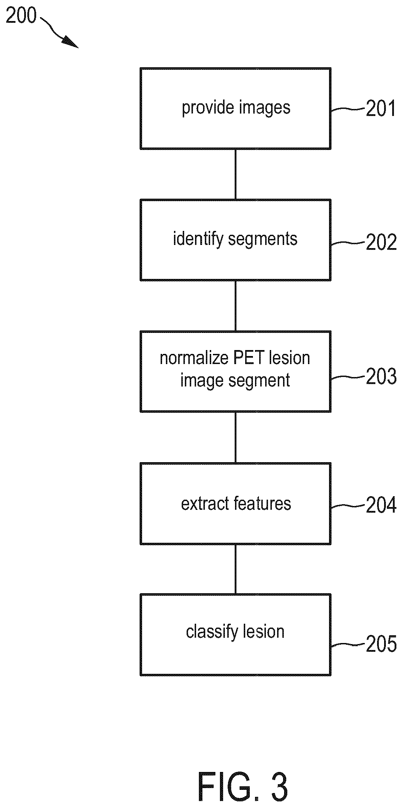

Fig. 3 shows schematically and exemplarily a method 200 for classifying lesions, the method comprising a step 201 of providing a dual-modal image of an imaging region, the dual-modal image comprising a CT image and a PET image registered to each other, wherein the imaging region includes a) a tissue region of interest comprising a lesion to be classified and b) a reference tissue region comprising healthy tissue, a step 202 of identifying, in each of the CT image and the PET image, a respective lesion image segment corresponding to the lesion in the tissue region of interest and, in the PET image, a reference image segment corresponding to the reference tissue region. The method 200 further comprises a step 203 of normalizing the lesion image segment in the PET image with respect to the reference image segment in the PET image, a step 204 of extracting values of a set of image features associated with the lesion from the lesion image segment in the CT image and from the normalized lesion image segment in the PET image, and a step 205 of classifying the lesion according to its severity based on the extracted image feature values.

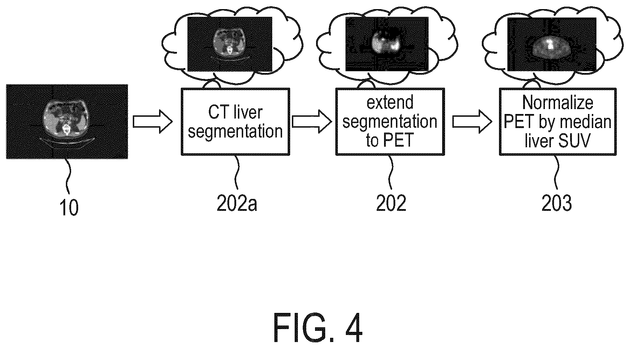

Fig. 4 shows schematically and exemplarily PET SUV normalization using CT liver segmentation. From a CT image 10 showing an imaging region comprising a patient's upper body (cf. first image in Fig. 4 as seen from left to right), the liver is segmented, in a step 202a, by application of a machine learning architecture as described, for instance, with respect to Fig. 2 above, which might also be regarded as a deep learning model. The CT image 10 with the liver segmented is shown in the second image of Fig. 4 as seen from left to right. The CT liver segmentation is then extended, in a step 202, to the PET image registered to the CT image 10 via the registration, as shown in the third image of Fig. 4 as seen from left to right. Hence, in the embodiment shown in Fig. 4, in each of the CT image 10 and the PET image, a respective reference image segment corresponding to the reference tissue region, in this case the liver, is identified, wherein the reference image segment in the PET image is identified as the image segment registered to the reference image segment identified, by application of the identifying machine learning architecture, in the CT image. Lastly, the PET image is normalized in terms of its SUVs with respect to the median SUV in the reference image segment identified in the PET image as corresponding to the liver, which results in a normalized intensity of the PET image, as shown in the last image of Fig. 4 as seen from left to right. In this case, the whole PET image is normalized, thereby particularly normalizing the lesion image segment, here corresponding to a prostate tumor region, which corresponds to step 203.

Preferably, for classifying the lesion, a machine learning architecture suitable for receiving image features values as an input and determining an associated lesion severity class as an output is used. The classifying unit 105 preferably comprises this machine learning architecture, which might be a random forest as described further above. The machine learning architecture used for classifying lesions and the set of image features provided to the machine learning architecture as an input for classifying the lesions are preferably determined in a joint determination process.

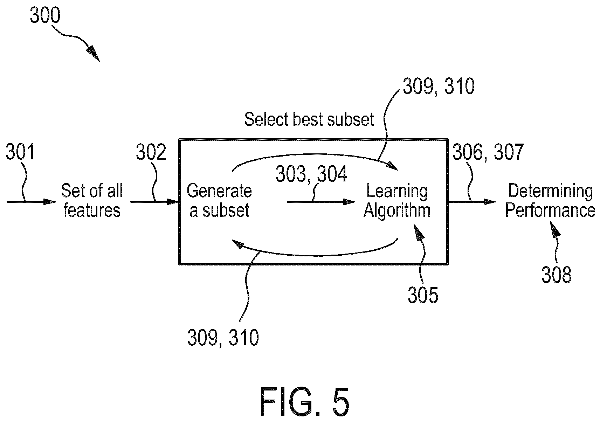

Fig. 5 illustrates schematically and exemplarily parts of such a joint determination process as an embodiment of a method 300 for choosing a set of image features and a machine learning architecture for use by the system 100 or in the method 200 for classifying lesions. The method 300 comprises a step 301 of providing a set of dual-modal training images, each training image comprising a CT image and a PET image registered to each other, in each of which a respective lesion image segment corresponding to a respective lesion in a tissue region of interest has been identified, wherein, for each of the regions in the training images, an assigned severity class is provided. In a further step 302 of the method 300, a candidate set of image features is chosen from a predefined collection of possible image features. The chosen candidate set of image features is therefore a subset generated from the set of all possible image features, as indicated in Fig. 5. The method 300 further comprises a step 303 of generating a training data set by extracting, from each of the lesion image segments identified in the training images, corresponding values of each of the candidate set of image features and assigning them to the respective severity class assigned to the lesion in the respective training image. In a further step 304 of the method 300, a candidate machine learning architecture suitable for receiving image feature values as an input and determining an associated lesion severity class as an output is chosen. Hence, for instance, a random forest with particular hyperparameters is chosen as a candidate machine learning architecture comprised by the classifying unit of the system 100. In a further step 305 of the method 300, the candidate machine learning architecture is trained on the training data set. The training of the candidate machine learning architecture may also be viewed as a learning algorithm, as indicated in Fig. 5. In a further step 306 of the method 300, a set of dual-modal test images is provided, wherein each test image comprises a CT image and a PET image, in each of which a respective lesion image segment corresponding to a respective lesion in a tissue region of interest has been identified, wherein, for each of the lesions in the test images, an assigned severity class is provided. The severity classes provided for the lesions in the test images may be understood as a ground truth for the purpose of testing. A test dataset is generated in a further step 307 of the method 300 by extracting, from each of the lesion image segments identified in the test images, corresponding values of each of the candidate set of image features and assigning them to the respective severity class assigned to the lesion in the respective test image. According to a further step 308 of the method 300, as indicated in Fig. 5, a performance of the candidate machine learning architecture on the test data set is determined based on a relation between the assigned severity classes provided for the lesions in the test images and the corresponding lesion severity classes determined by the candidate machine learning architecture. The performance may be measured, for instance, in terms of an accuracy, a sensitivity, a specificity of the candidate machine learning architecture and/or a speed at which the candidate machine learning architecture has arrived at the classification, based on the candidate set of image features.

The above described steps 301 to 308 of the method 300 are, in a step 309, repeated with increasing numbers of image features in the candidate set of image features, wherein for each repetition, the candidate set of image features is enlarged by a further image feature from the predefined collection of possible image feature, the further image feature being the one that results in the highest increase in performance, until an abort criterion is met. Hence, for a fixed candidate machine learning architecture, a growing set of image features to be extracted is used until an abort criterion is met, which is indicated by the cycle in Fig. 5. The abort criterion might be that the number of candidate image features to be extracted has grown above a predefined threshold, or it might indicate that the increase in performance has fallen below a predefined threshold. What is not explicitly shown in Fig. 5 is that all previous steps, i.e. steps 301 to 309 can then be repeated in a further step x) of the method 300 with a plurality of candidate machine learning architectures, i.e., for instance, with a random forest with different hyperparameters. The set of image features and the machine learning architecture to be used for classifying lesions are then chosen from the respective candidates according to the performances determined for the respective combinations, i.e. pairs.

Hence, after concatenation and standardization of the image features relating to the CT and the PET images, not all image features are used for model training. In order to reduce overfitting, complexity, training time and in order to arrive at a stable and generalized model, feature selection is carried out before model training. For selecting the most important features, sequential feature selection (SFS) can be applied, i.e., for instance, at the beginning of each of the cycles indicated in Fig. 5. In the SFS method, one starts with an empty set of image features and then adds, one by one, the features performing best in improving classification results with already selected features. In each iteration, the image feature that gives a maximum improvement is included in the set of selected features. A pseudocode followed in an SFS method might be expressed as follows:

In a first step, create an empty set

$$Y_k = \{\emptyset\}, \quad k = 0 \,. \qquad (1)$$

In a second step, select the best remaining feature

$$x^+ = \arg\max_{x \in Y \setminus Y_k} [J(Y_k \cup \{x\})] \,. \qquad (2)$$

And in a third step,

$$\text{if } J(Y_k \cup \{x^+\}) > J(Y_k) \,, \qquad (3)$$

   a.

$$\text{update } Y_{k+1} = Y_k \cup \{x^+\} \,, \qquad (4a)$$

   b.

$$\text{increase } k \to k + 1, \qquad (4b)$$

c.

$$\text{go back to step 2,} \quad (4c)$$

wherein Y refers to the set of all possible, i.e. the predefined collection of image features, and *J* measures a performance of the respective machine learning architecture using the subset of image features in its argument.

The candidate machine learning architectures considered for classification may include logistic regression, support vector machines (SVMs), ensemble methods like random forest, XGBoost and balanced random forests, particularly for a classification into indolent and aggressive prostate cancer. The performance of the candidate machine learning architectures may be estimated using five-fold cross-validation, for instance. Hence, the test images may include different subsets of the training images, thereby performing cross-validation. In five-fold cross-validation, five different subsets of the training images are chosen to form the test images.

In a preferred embodiment, the plurality of candidate machine learning architectures correspond to random forests with different hyperparameters, the hyperparameters being chosen successively according to a grid search. This allows for a relatively low complexity while still showing a very good performance on cross-validation. According to the grid search, the hyperparameters of the random forest may be tuned, for instance, using the sklearn library. Possible hyperparameter values resulting from a grid search are shown in below Table 3.

Table 3: Tuned hyperparameters for random forest model

| Hyperparameter | Value |
|---|---|
| Splitting criterion | entropy |
| maximum tree depth | 5 |
| maximum number of features considered for each split | 2 |
| maximum number of trees (estimators) | 500 |

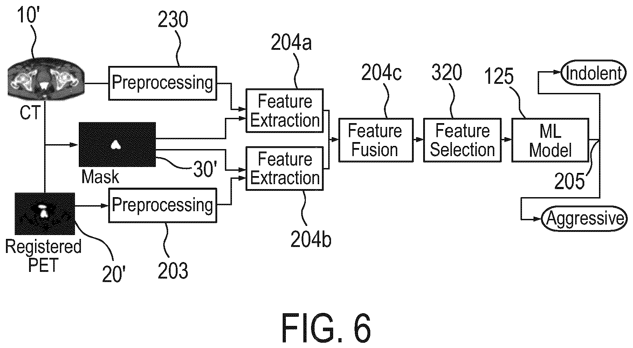

Fig. 6 illustrates schematically and exemplarily embodiments according to several of the described aspects. It is shown that a CT image 10' and a PET image 20' of a patient's upper body are provided, which might be viewed as a single dual image. In this dual image, a lesion image segment corresponding the patient's prostate is identified, thereby generating a mask 30' for the dual image. The CT image 10' and the PET image 20' are both preprocessed, wherein the preprocessing 230, which might also be viewed as a modification, of the CT image 10' may refer a clipping of the CT image values to a range, or window, including image values corresponding to a reference tissue region and image values corresponding to a tissue region of interest, and a subsequent mapping of the range, or window, to a full range of possible CT image values, i.e., for instance, intensities. The reference tissue region may particularly correspond to the patient's liver, and the tissue region of interest may refer to the patient's prostate. The preprocessing 203 of the PET image 20' may refer to a normalization of the PET image values with respect to a reference image segment identified in the PET image 20' and corresponding to the reference tissue region, i.e. particularly the patient's liver.

As indicated in Fig. 6 by the blocks 204a, 204b and 204c, CT image features are extracted from the preprocessed CT image 10' (block 204a) and PET image features are extracted from the preprocessed PET image 20' (block 204b), wherein the extracted CT image features and the extracted PET image features are then fused to a joint set of dual, CT/PET image features (block 204c). These steps amount, in conjunction, to an extraction 204 of the joint set of dual, CT/PET image features.

Block 320 shown in Fig. 6 indicates that not all of the previously extracted image features are used for classification, but only a selected subset of them. Hence, while with reference to Figs. 4 and 5 embodiments were described according to which a selection of image features to be used for classification is made before the extraction of their values from the images, in other embodiments values of all possible, or at least a predefined, possibly relatively large, collection of image features is extracted from the images, wherein only the values of a selected subset of these image features is then forwarded as an input for the classifying unit, particularly its machine learning architecture.

The machine learning architecture comprised by the classification unit, which might also be regarded as a machine learning model, is indicated in Fig. 6 by block 125. In the embodiment shown, it classifies, in terms of its output, the lesions shown in the CT and the PET images and masked by the mask 30' according to a binary classification into indolent and aggressive lesions, wherein, in particular, the lesions can be prostate tumor regions.

The classification performance of the system has been measured, for an embodiment in which prostate tumors are classified into indolent and aggressive tumors and the liver is chosen as the reference tissue region, using several performance measures and both for five-fold cross-validation using the training data as well as using separate test data. It is to be noted that the training data and the test data may refer to the training images and the test images, respectively, or to the image features extracted from the training images and the test images, respectively. The performances achieved in this particular embodiment are shown in below Table 4.

Table 4: Results for indolent vs. aggressive prostate tumor classification

| Data | Samples | Accuracy | $F_1$ score | Precision | Sensitivity | Specificity | ROC-AUC |
|---|---|---|---|---|---|---|---|
| Five-fold cross-validation | 113 | 0.89 | 0.90 | 0.85 | 0.96 | 0.81 | 0.94 |
| Test | 30 | 0.9 | 0.87 | 0.846 | 0.916 | 0.88 | 0.90 |

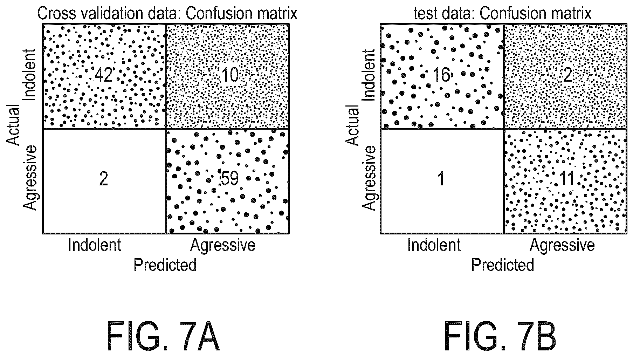

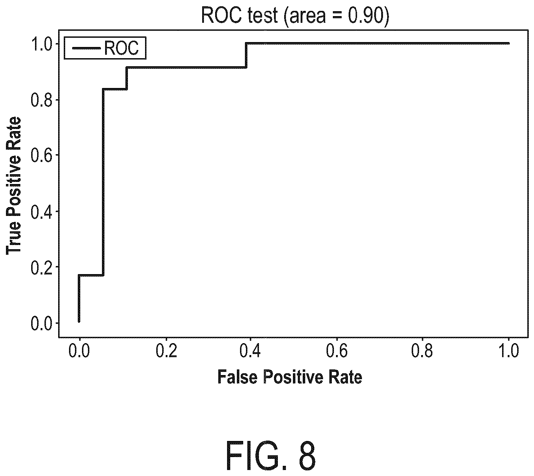

Figs. 7A and 7B show confusion matrices corresponding to the performance of the system in the above embodiment, wherein the confusion matrix in Fig. 7A relates to a validation in terms of five-fold cross-validation and Fig. 7B relates to a validation in terms of separate test data. As can be seen from above Table 4 and Figs. 7A and 7B, the risk of confusion, i.e. the risk that the system produces false negative or false positive classifications, is relatively low. In particular, the risk for erroneously classifying an actually aggressive prostate tumor region as an indolent tumor region, i.e. the false negative rate, which is clinically particularly relevant, has been found to be low for both validation schemes. Fig. 8 shows the receiver operator characteristic (ROC) curve for the embodiment to which also Table 4 and Figs. 7A and 7B relate in the case of using test data for validation, wherein the area under this curve (ROC-AUC) has been determined to be 0.90.

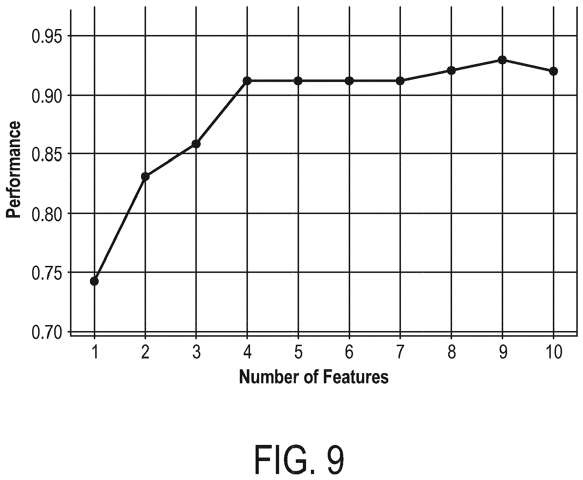

In Fig. 9, the dependence of the classification accuracy on the increasing number of image features selected to be used for classification is shown for the case of cross-validation. It can be appreciated from Fig. 9 that the performance, as measured in terms of classification accuracy, increases relatively rapidly initially, i.e. once the first few, most important image features are added. At the same time, the increase in performance becomes less as a growing number of features, with decreasing importance, are added. The accuracy might even drop although a further image feature is being added, as can also be seen in Fig. 9. Nevertheless, it can be tolerable or even favorable to use such a further image feature, since this might lead to a generalization of the model.

Embodiments disclosed herein relate to evaluating Gleason grade scores for prostate cancer. Gleason grading is the most commonly used tool to determine prostate cancer prognosis. However, Gleason grading is a manual process whereby a pathologist manually assesses multiple biopsy samples under a microscope, and assigns a grade to the cancer based on its severity. A machine learning based model is therefore proposed in an embodiment to automatically classify tumor habitats in $^{68}$Ga-PSMA PET/CT images with Gleason grade groups (indolent vs. aggressive). The present disclosure hence relates to an artificial intelligence model for mapping, in particular, Gleason grade scores to PSMA-PET/CT scans. Gleason grading capabilities are added to PSMA-PET/CT scanning for tumor habitats with the intent to classify them as aggressive or indolent type. Tagging habitats with Gleason grades to categorize them as aggressive or indolent type helps in biopsy planning to extract the right tissue samples and tagging helps to target aggressive tumors during the radiation therapy. The model can be trained by learning from the ground truth of cancer regions (ROIs) classified with Gleason grade groups of PET and CT imaging data. The ground truth Gleason grades were classified by doctors after going through biopsy samples. This enables the extraction of the correct biopsy samples, and also the targeting of aggressive tumors during therapy.

The proposed systems and methods address the following challenges and opportunities in existing systems and methods: 1) SUV values vary across multiple vendors based on the detector type, physiological conditions, acquisition and reconstruction parameters, 2) tagging habitats with Gleason grade to categorize them as aggressive or indolent type helps to target the right tissue for biopsy, 3) prostate biopsies incur the risk of being false-negative based on the location of samples, and this might affect the cancer grading, 4) PSMA-PET/CT imaging potentially provides superior detection of prostate cancer lesions with better sensitivity than multi-parametric MRI.

It is proposed to normalize (e.g., using an adapted VGG-16 neural network) PSMA SUV values with respect to liver tissue, particularly a corresponding median SUV, for supporting multi-vendor and machine variants, which is based on different scintillating crystals materials (LSO, BGO, LYSO) used in the machine. PET normalization also helps to normalize SUV values that will vary with respect to the physiological condition of a patient and the uptake time of the nuclear tracer. Multi-modal fusion of hand-crafted features from CT and PET images, such as radiomics features extracted from an ROI bounding box, is proposed, as well as a machine learning model to map Gleason scores, and to classify habitat tumors into aggressive vs. indolent ones. Concatenated features from PET and CT gave the best results. Various machine learning models were tried to classify indolent and aggressive prostate cancer with a good performance, particularly random forests. To add Gleason grading capabilities to PSMA-PET/CT scanning for tumor habitats, a process as captured, for instance, in above Fig. 6 can be followed. In particular, the process can involve a pre-processing, habitat ROI mask generation (either by a user or in an automatic way), multi-modal feature extraction, feature selection and fusion, and a machine learning model to classify indolent and aggressive type tumor habitats.

Although in the above described embodiments, the dual-modal images each comprise a CT and a PET image, more generally, for instance, the dual-modal images might each comprise a CT and a functional image, wherein the functional image might also be different from a PET image. Moreover, although in many embodiments described above the tissue region of interest is or lies in a patient's prostate and the reference tissue region is or lies in the patient's liver, any other combination of tissue regions of interest and reference tissue regions is generally possible.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the providing of the dual image, the identifying of the lesion image segments and the reference image segments, the normalizing of the lesion image segment in the PET image, the extracting of image feature values and the classifying of lesions, et cetera, performed by one or several units or devices can be performed by any other number of units or devices. These procedures can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

A system for classifying lesions is provided that comprises an image providing unit for providing a dual-modal image of an imaging region, the dual-modal image comprising a CT image and a registered PET image, the imaging region including a tissue region of interest comprising a lesion and a reference tissue region. The system further comprises an identifying unit for identifying, in the CT and the PET image, a respective lesion image segment corresponding to the lesion and, in the PET image, a reference image segment corresponding to the reference tissue region. Moreover, the system comprises a normalizing unit for normalizing the lesion image segment in the PET image with respect to the reference image segment, an image feature extracting unit for extracting values for a set of image features from both lesion image segments, and a classifying unit for classifying the lesion based on the extracted values.

**Claims**

1. System (100) for classifying lesions, comprising:

   - an image providing unit (101) for providing a dual-modal image of an imaging region, the dual-modal image comprising a computed tomography image (10, 10') and a positron emission tomography image (20') registered to each other, wherein the imaging region includes a) a tissue region of interest comprising a lesion to be classified and b) a reference tissue region comprising healthy tissue,
   - an identifying unit (102) for identifying, in each of the computed tomography image (10, 10') and the positron emission tomography image (20'), a respective lesion image segment corresponding to the lesion in the tissue region of interest and, in the positron emission tomography image (20'), a reference image segment corresponding to the reference tissue region,
   - a normalizing unit (103) for normalizing the lesion image segment in the positron emission tomography image (20') with respect to the reference image segment in the positron emission tomography image (20'),
   - an image feature extracting unit (104) for extracting values of a set of image features associated with the lesion from the lesion image segment in the computed tomography image (10, 10') and from the normalized lesion image segment in the positron emission tomography image (20'), and
   - a classifying unit (105) for classifying the lesion according to its severity based on the extracted image feature values.

2. System (100) according to claim 1, wherein the identifying unit (102) is adapted to identify, in each of the computed tomography image (10, 10') and the positron emission tomography image (20'), a respective reference image segment corresponding to the reference tissue region, wherein the reference image segment in the positron emission tomography image (20') is identified as the image segment registered to the reference image segment identified in the computed tomography image (10, 10').

3. System (100) according to any of the preceding claims, wherein the tissue region of interest refers to a patient's prostate, the lesion is a prostate tumor region and the positron emission tomography image (20') is generated using an imaging substance binding to prostate specific membrane antigen.

4. System (100) according to claim 3, wherein the lesion is classified according to a binary classification of severity based on a Gleason score, the binary classification distinguishing indolent from aggressive prostate tumor regions.

5. System (100) according to claims 1 or 2, wherein the reference tissue region is a patient's liver, or according to claims 3 or 4, wherein the reference tissue region is the patient's liver.

6. System (100) according to any of the preceding claims, wherein the set of image features includes, as features whose values are to be extracted from the lesion image segment in the computed tomography image (10, 10'), a total energy, a maximum two-dimensional diameter in row-direction, a sphericity, a surface area, a large area emphasis and a dependence entropy, and, as features whose values are to be extracted from the normalized lesion image segment in the positron emission tomography image (20'), a 90th percentile, a minimum, a flatness and a sphericity.

7. System (100) according to claim 2 or any of claims 3 to 6 when referring to claim 2, wherein the identifying unit (102) comprises, for identifying the reference image segment in the computed tomography image (10, 10'), a machine learning architecture adapted to receive a computed tomography image (10, 10') of the imaging region including the reference tissue region as an input and to determine the reference image segment in the computed tomography as an output.

8. System (100) according to claim 7, wherein the machine learning architecture comprised by the identifying unit (102) comprises a convolutional neural network with convolutional, activation and pooling layers as a base network (115) and, at a plurality of convolutional stages of the base network (115), a respective side set of convolutional layers connected to a respective end of the convolutional layers of the base network (115) at the respective convolutional stage, wherein the reference image segment in the computed tomography image (10, 10') is determined based on outputs of the plurality of side sets of convolutional layers.

9. System (100) according to any of the preceding claims, wherein the classifying unit (105) comprises, for classifying the lesion, a machine learning architecture (125) suitable for receiving image feature values as an input and determining an associated lesion severity class as an output.

10. A computer-implemented method (200) for classifying lesions, comprising:

   - providing (201) a dual-modal image of an imaging region, the dual-modal image comprising a computed tomography image (10, 10') and a positron emission tomography image (20') registered to each other, wherein the imaging region includes a) a tissue region of interest comprising a lesion to be classified and b) a reference tissue region comprising healthy tissue,
   - identifying (202), in each of the computed tomography image (10, 10') and the positron emission tomography image (20'), a respective lesion image segment corresponding to the lesion in the tissue region of interest and, in the positron emission tomography image (20'), a reference image segment corresponding to the reference tissue region,
   - normalizing (203) the lesion image segment in the positron emission tomography image (20') with respect to the reference image segment in the positron emission tomography image (20'),
   - extracting (204; 204a, 204b) values of a set of image features associated with the lesion from the lesion image segment in the computed tomography image (10, 10') and from the normalized lesion image segment in the positron emission tomography image (20'), and
   - classifying (205) the lesion according to its severity based on the extracted image feature values.

11. A computer-implemented method (300) for choosing a set of image features and a machine learning architecture for use by a system (100) according to claim 1 or in a method (200) according to claim 10 for classifying lesions, comprising:

   - providing (301) a set of dual-modal training images, each training image comprising a computed tomography image (10, 10') and a positron emission tomography image (20') registered to each other, in each of which a respective lesion image segment corresponding to a respective lesion in a tissue region of interest has been identified, wherein, for each of the lesions in the training images, an assigned severity class is provided,

- choosing (302) a candidate set of image features from a predefined collection of possible image features,
- generating (303) a training dataset by extracting, from each of the lesion image segments identified in the training images, corresponding values of each of the candidate set of image features and assigning them to the respective severity class assigned to the lesion in the respective training image,
- choosing (304) a candidate machine learning architecture suitable for receiving image feature values as an input and determining an associated lesion severity class as an output,
- training (305) the candidate machine learning architecture on the training dataset,
- providing (306) a set of dual-modal test images, each test image comprising a computed tomography image (10, 10') and a positron emission tomography image (20'), in each of which a respective lesion image segment corresponding to a respective lesion in a tissue region of interest has been identified, wherein, for each of the lesions in the test images, an assigned severity class is provided,
- generating (307) a test dataset by extracting, from each of the lesion image segments identified in the test images, corresponding values of each of the candidate set of image features and assigning them to the respective severity class assigned to the lesion in the respective test image,
- determining (308) a performance of the candidate machine learning architecture on the test dataset based on a relation between the assigned severity classes provided for the lesions in the test images and the corresponding lesion severity classes determined by the candidate machine learning architecture,
- repeating (309) the above steps with increasing numbers of image features in the candidate set of image features, wherein for each repetition, the candidate set of image features is enlarged by a further image feature from the predefined collection of possible image features, the further image feature being the one that results in the highest increase in performance, until an abort criterion is met, and
- repeating (310) the above steps with at least one candidate machine learning architecture, wherein the set of image features and the machine learning architecture to be used for classifying lesions are chosen from the respective candidates according to the determined performances.

12. Method according to claim 11, wherein the plurality of candidate machine learning architectures correspond to random forests with different hyperparameters.

13. Method according to claim 12, wherein the hyperparameters are chosen successively according to a grid search.

14. Method according to any of claims 11 to 13, wherein the test images include different subsets of the training images, thereby performing cross-validation.

15. Computer program for classifying lesions, the program comprising program code means for causing a system according to any of claims 1 to 9 to execute the method according to claim 10 when the program is run on a computer controlling the system.

**Patentansprüche**

1. System (100) zum Klassifizieren von Läsionen, umfassend:

- eine Bildbereitstellungseinheit (101) zum Bereitstellen eines dual-modalen Bildes eines Bildgebungsbereichs, wobei das dual-modale Bild ein Computertomographiebild (10, 10') und ein Positronenemissionstomographiebild (20') umfasst, die zueinander registriert sind, wobei der Bildgebungsbereich a) einen Gewebebereich von Interesse, der eine zu klassifizierende Läsion umfasst, und b) einen Referenzgewebebereich, der gesundes Gewebe umfasst, beinhaltet,
- eine Identifizierungseinheit (102) zum Identifizieren eines jeweiligen Läsionsbildsegments, das der Läsion im Gewebebereich von Interesse entspricht, in jedem von dem Computertomographiebild (10, 10') und dem Positronenemissionstomographiebild (20'), und eines Referenzbildsegments, das dem Referenzgewebebereich entspricht, in dem Positronenemissionstomographiebild (20'),
- eine Normalisierungseinheit (103) zum Normalisieren des Läsionsbildsegments in dem Positronenemissionstomographiebild (20') in Bezug auf das Referenzbildsegment in dem Positronenemissionstomographiebild (20'),
- eine Bildmerkmal-Extraktionseinheit (104) zum Extrahieren von Werten eines Satzes von Bildmerkmalen, die mit der Läsion verknüpft sind, aus dem Läsionsbildsegment in dem Computertomographiebild (10, 10') und aus dem normalisierten Läsionsbildsegment in dem Positronenemissionstomographiebild (20'), und
- eine Klassifizierungseinheit (105) zum Klassifizieren der Läsion nach ihrem Schweregrad basierend auf den extrahierten Bildmerkmalswerten.

2. System (100) nach Anspruch 1, wobei die Identifizierungseinheit (102) dazu angepasst ist, in jedem von dem Computertomographiebild (10, 10') und dem Positronenemissionstomographiebild (20') ein jeweiliges Referenzbildsegment zu identifizieren, das dem Referenzgewebebereich entspricht, wobei das Referenzbildsegment in dem Positronenemissionstomographiebild (20') als das Bildsegment identifiziert wird, das mit dem in dem Computertomographiebild (10, 10') identifizierten Referenzbildsegment registriert ist.

3. System (100) nach einem der vorstehenden Ansprüche, wobei sich der Gewebebereich von Interesse auf die Prostata eines Patienten bezieht, die Läsion ein Prostatatumorbereich ist und das Positronenemissionstomographiebild (20') unter Verwendung einer Bildgebungssubstanz erzeugt wird, die sich an prostataspezifisches Membranantigen bindet.

4. System (100) nach Anspruch 3, wobei die Läsion gemäß einer binären Klassifizierung eines Schweregrads basierend auf einem Gleason-Score klassifiziert wird, wobei die binäre Klassifizierung indolente von aggressiven Prostatatumorbereichen unterscheidet.

5. System (100) nach Ansprüchen 1 oder 2, wobei der Referenzgewebebereich die Leber eines Patienten ist, oder nach Ansprüchen 3 oder 4, wobei der Referenzgewebebereich die Leber des Patienten ist.

6. System (100) nach einem der vorstehenden Ansprüche, wobei der Satz von Bildmerkmalen als Merkmale, deren Werte aus dem Läsionsbildsegment in dem Computertomographiebild (10, 10') zu extrahieren sind, eine Gesamtenergie, einen maximalen zweidimensionalen Durchmesser in Zeilenrichtung, eine Sphärizität, einen Oberflächenbereich, eine Großflächenbetonung und eine Abhängigkeitsentropie, und als Merkmale, deren Werte aus dem normalisierten Läsionsbildsegment in dem Positronenemissionstomographiebild (20') zu extrahieren sind, ein 90. Perzentil, ein Minimum, eine Ebenheit und eine Sphärizität beinhaltet.

7. System (100) nach Anspruch 2 oder einem der Ansprüche 3 bis 6, wenn auf Anspruch 2 Bezug genommen wird, wobei die Identifizierungseinheit (102) zum Identifizieren des Referenzbildsegments in dem Computertomographiebild (10, 10') eine Maschinenlernarchitektur umfasst, die dazu angepasst ist, ein Computertomographiebild (10, 10') des Bildgebungsbereichs, der den Referenzgewebebereich beinhaltet als eine Eingabe zu empfangen und um das Referenzbildsegment in der Computertomographie als eine Ausgabe zu bestimmen.

8. System (100) nach Anspruch 7, wobei die von der Identifizierungseinheit (102) umfasste Maschinenlernarchitektur ein neuronales Faltungsnetzwerk mit Faltungs-, Aktivierungs- und Pooling-Schichten als ein Basisnetzwerk (115) und in einer Vielzahl von Faltungsstufen des Basisnetzwerks (115) einen jeweiligen Seitensatz von Faltungsschichten umfasst, der mit einem jeweiligen Ende der Faltungsschichten des Basisnetzwerks (115) in der jeweiligen Faltungsstufe verbunden ist, wobei das Referenzbildsegment in dem Computertomographiebild (10, 10') basierend auf Ausgaben der Vielzahl von Seitensätzen von Faltungsschichten bestimmt wird.

9. System (100) nach einem der vorstehenden Ansprüche, wobei die Klassifizierungseinheit (105) zum Klassifizieren der Läsion eine Maschinenlernarchitektur (125) umfasst, die zum Empfangen von Bildmerkmalswerten als eine Eingabe und Bestimmen einer verknüpften Läsionsschweregradklasse als eine Ausgabe geeignet ist.

10. Computerimplementiertes Verfahren (200) zum Klassifizieren von Läsionen, umfassend:

   - Bereitstellen (201) eines dual-modalen Bildes eines Bildgebungsbereichs, wobei das dual-modale Bild ein Computertomographiebild (10, 10') und ein Positronenemissionstomographiebild (20') umfasst, die zueinander registriert sind, wobei der Bildgebungsbereich a) einen Gewebebereich von Interesse, der eine zu klassifizierende Läsion umfasst, und b) einen Referenzgewebebereich, der gesundes Gewebe umfasst, beinhaltet,
   - Identifizieren (202) eines jeweiligen Läsionsbildsegments, das der Läsion im Gewebebereich von Interesse entspricht, in jedem von dem Computertomographiebild (10, 10') und dem Positronenemissionstomographiebild (20'), und eines Referenzbildsegments, das dem Referenzgewebebereich entspricht, in dem Positronenemissionstomographiebild (20'),
   - Normalisieren (203) des Läsionsbildsegments in dem Positronenemissionstomographiebild (20') in Bezug auf das Referenzbildsegment in dem Positronenemissionstomographiebild (20'),
   - Extrahieren (204; 204a, 204b) von Werten eines Satzes von Bildmerkmalen, die mit der Läsion verknüpft sind, aus dem Läsionsbildsegment in dem Computertomographiebild (10, 10') und aus dem normalisierten Läsionsbildsegment in dem Positronenemissionstomographiebild (20'), und
   - Klassifizieren (205) der Läsion nach ihrem Schweregrad basierend auf den extrahierten Bildmerkmalswerten.

**11.** Computerimplementiertes Verfahren (300) zum Wählen eines Satzes von Bildmerkmalen und einer Maschinenlernarchitektur zur Verwendung durch ein System (100) nach Anspruch 1 oder in einem Verfahren (200) nach Anspruch 10 zum Klassifizieren von Läsionen, umfassend:

- Bereitstellen (301) eines Satzes von dual-modalen Trainingsbildern, wobei jedes Trainingsbild ein Computertomographiebild (10, 10') und ein Positronenemissionstomographiebild (20') umfasst, die zueinander registriert sind und in denen jeweils ein jeweiliges Läsionsbildsegment, das einer jeweiligen Läsion in einem Gewebebereich von Interesse entspricht, identifiziert worden ist, wobei für jede der Läsionen in den Trainingsbildern eine zugewiesene Schweregradklasse bereitgestellt wird,
- Wählen (302) eines Kandidatensatzes von Bildmerkmalen aus einer vordefinierten Sammlung möglicher Bildmerkmale,
- Erzeugen (303) eines Trainingsdatensatzes durch Extrahieren entsprechender Werte jedes von dem Kandidatensatz von Bildmerkmalen aus jedem der in den Trainingsbildern identifizierten Läsionsbildsegmente und Zuweisen dieser Werte zu der jeweiligen Schweregradklasse, die der Läsion in dem jeweiligen Trainingsbild zugewiesen ist,
- Wählen (304) einer Kandidaten-Maschinenlernarchitektur, die zum Empfangen von Bildmerkmalswerten als eine Eingabe und Bestimmen einer verknüpften Läsionsschweregradklasse als eine Ausgabe geeignet ist,
- Trainieren (305) der Kandidaten-Maschinenlernarchitektur an dem Trainingsdatensatz,
- Bereitstellen (306) eines Satzes von dual-modalen Testbildern, wobei jedes Testbild ein Computertomographiebild (10, 10') und ein Positronenemissionstomographiebild (20') umfasst, in denen jeweils ein jeweiliges Läsionsbildsegment, das einer jeweiligen Läsion in einem Gewebebereich von Interesse entspricht, identifiziert worden ist, wobei für jede der Läsionen in den Testbildern eine zugewiesene Schweregradklasse bereitgestellt wird,
- Erzeugen (307) eines Testdatensatzes durch Extrahieren entsprechender Werte jedes von dem Kandidatensatz von Bildmerkmalen aus jedem der in den Testbildern identifizierten Läsionsbildsegmente und Zuweisen dieser Werte zu der jeweiligen Schweregradklasse, die der Läsion in dem jeweiligen Testbild zugewiesen ist,
- Bestimmen (308) einer Leistung der Kandidaten-Maschinenlernarchitektur an dem Testdatensatz basierend auf einer Beziehung zwischen den zugewiesenen Schweregradklassen, die für die Läsionen in den Testbildern bereitgestellt werden, und den entsprechenden Läsionsschweregradklassen, die durch die Kandidaten-Maschinenlernarchitektur bestimmt werden,
- Wiederholen (309) der obigen Schritte mit zunehmender Anzahl von Bildmerkmalen im Kandidatensatz von Bildmerkmalen, wobei der Kandidatensatz von Bildmerkmalen bei jeder Wiederholung um ein weiteres Bildmerkmal aus der vordefinierten Sammlung möglicher Bildmerkmale erweitert wird, wobei das weitere Bildmerkmal dasjenige ist, das zum höchsten Leistungsanstieg führt, bis ein Abbruchkriterium erfüllt ist, und
- Wiederholen (310) der obigen Schritte mit mindestens einer Kandidaten-Maschinenlernarchitektur, wobei der Satz von Bildmerkmalen und die Maschinenlernarchitektur, die zum Klassifizieren von Läsionen zu verwenden sind, aus den jeweiligen Kandidaten entsprechend den bestimmten Leistungen gewählt werden.

**12.** Verfahren nach Anspruch 11, wobei die Vielzahl von Kandidaten-Maschinenlernarchitekturen Zufallswäldern mit unterschiedlichen Hyperparametern entsprechen.

**13.** Verfahren nach Anspruch 12, wobei die Hyperparameter nacheinander gemäß einer Rastersuche gewählt werden.

**14.** Verfahren nach einem der Ansprüche 11 bis 13, wobei die Testbilder unterschiedliche Teilmengen der Trainingsbilder beinhalten, und dadurch eine Kreuzvalidierung durchgeführt wird.

**15.** Computerprogramm zum Klassifizieren von Läsionen, wobei das Programm Programmcodemittel zum Veranlassen eines Systems nach einem der Ansprüche 1 bis 9 umfasst, das Verfahren nach Anspruch 10 auszuführen, wenn das Programm auf einem Computer läuft, der das System steuert.

**Revendications**

**1.** Système (100) de classification de lésions, comprenant :

- une unité de fourniture d'image (101) pour fournir une image bimodale d'une région d'imagerie, l'image bimodale comprenant une image de tomodensitométrie (10, 10') et une image de tomographie par émission de positons (20') recalées l'une par rapport à l'autre, dans lequel la région d'imagerie inclut a) une région tissulaire d'intérêt comprenant une lésion à classer et b) une région tissulaire de référence comprenant un tissu sain,
- une unité d'identification (102) pour identifier, dans chacune parmi l'image de tomodensitométrie (10, 10') et l'image de tomographie par émission de positons (20'), un segment d'image de lésion respectif correspondant à la lésion dans la région tissulaire d'intérêt et, dans l'image de tomographie par émission de positons (20'), un segment d'image de référence correspondant à la région tissulaire de référence,
- une unité de normalisation (103) pour normaliser le segment d'image de lésion dans l'image de tomographie par émission de positons (20') par rapport au segment d'image de référence dans l'image de tomographie par émission de positons (20'),
- une unité d'extraction de caractéristiques d'image (104) pour extraire des valeurs d'un ensemble de caractéristiques d'image associées à la lésion à partir du segment d'image de lésion dans l'image de tomodensitométrie (10, 10') et à partir du segment d'image de lésion normalisé dans l'image de tomographie par émission de positons (20'), et
- une unité de classification (105) pour classer la lésion en fonction de sa gravité sur la base des valeurs de caractéristiques d'image extraites.

2. Système (100) selon la revendication 1, dans lequel l'unité d'identification (102) est adaptée pour identifier, dans chacune parmi l'image de tomodensitométrie (10, 10') et l'image de tomographie par émission de positons (20'), un segment d'image de référence respectif correspondant à la région tissulaire de référence, dans lequel le segment d'image de référence dans l'image de tomographie par émission de positons (20') est identifié comme le segment d'image recalé par rapport au segment d'image de référence identifié dans l'image de tomodensitométrie (10, 10').

3. Système (100) selon l'une quelconque des revendications précédentes, dans lequel la région tissulaire d'intérêt fait référence à la prostate d'un patient, la lésion est une région tumorale de la prostate et l'image de tomographie par émission de positons (20') est générée à l'aide d'une substance d'imagerie se liant à l'antigène membranaire spécifique de la prostate.

4. Système (100) selon la revendication 3, dans lequel la lésion est classée selon une classification binaire de gravité basée sur un score de Gleason, la classification binaire distinguant les régions tumorales indolentes des régions tumorales agressives de la prostate.

5. Système (100) selon la revendication 1 ou 2, dans lequel la région tissulaire de référence est le foie d'un patient, ou selon la revendication 3 ou 4, dans lequel la région tissulaire de référence est le foie du patient.

6. Système (100) selon l'une quelconque des revendications précédentes, dans lequel l'ensemble de caractéristiques d'image inclut, en tant que caractéristiques dont les valeurs doivent être extraites du segment d'image de lésion dans l'image de tomodensitométrie (10, 10'), une énergie totale, un diamètre bidimensionnel maximal dans la direction des lignes, une sphéricité, une surface, une grande accentuation de surface et une entropie de dépendance, et, en tant que caractéristiques dont les valeurs doivent être extraites du segment d'image de lésion normalisé dans l'image de tomographie par émission de positons (20'), un $90^{ième}$ percentile, un minimum, une planéité et une sphéricité.

7. Système (100) selon la revendication 2 ou l'une quelconque des revendications 3 à 6 lorsqu'elle se rapporte à la revendication 2, dans lequel l'unité d'identification (102) comprend, pour identifier le segment d'image de référence dans l'image de tomodensitométrie (10, 10'), une architecture d'apprentissage automatique adaptée pour recevoir une image de tomodensitométrie (10, 10') de la région d'imagerie incluant la région tissulaire de référence en tant qu'entrée et pour déterminer le segment d'image de référence dans la tomodensitométrie en tant que sortie.

8. Système (100) selon la revendication 7, dans lequel l'architecture d'apprentissage automatique constituée par l'unité d'identification (102) comprend un réseau neuronal convolutif doté de couches convolutives, d'activation et de regroupement comme réseau de base (115) et, à une pluralité d'étages convolutifs du réseau de base (115), un ensemble latéral respectif de couches convolutives reliées à une extrémité respective des couches convolutives du réseau de base (115) à l'étage convolutif respectif, dans lequel le segment d'image de référence dans l'image de tomodensitométrie (10, 10') est déterminé sur la base des sorties de la pluralité d'ensembles latéraux de couches convolutives.

9. Système (100) selon l'une quelconque des revendications précédentes, dans lequel l'unité de classification (105) comprend, pour classer la lésion, une architecture d'apprentissage automatique (125) appropriée pour recevoir des valeurs de caractéristiques d'image en tant qu'entrée et déterminer une classe de gravité de lésion associée en tant que sortie.

**10.** Procédé implémenté par ordinateur (200) de classification de lésions, comprenant :

- la fourniture (201) d'une image bimodale d'une région d'imagerie, l'image bimodale comprenant une image de tomodensitométrie (10, 10') et une image de tomographie par émission de positons (20') recalées l'une par rapport à l'autre, dans lequel la région d'imagerie inclut a) une région tissulaire d'intérêt comprenant une lésion à classer et b) une région tissulaire de référence comprenant un tissu sain,
- l'identification (202), dans chacune parmi l'image de tomodensitométrie (10, 10') et l'image de tomographie par émission de positons (20'), d'un segment d'image de lésion respectif correspondant à la lésion dans la région tissulaire d'intérêt et, dans l'image de tomographie par émission de positons (20'), d'un segment d'image de référence correspondant à la région tissulaire de référence,
- la normalisation (203) du segment d'image de lésion dans l'image de tomographie par émission de positons (20') par rapport au segment d'image de référence dans l'image de tomographie par émission de positons (20'),
- l'extraction (204 ; 204a, 204b) de valeurs d'un ensemble de caractéristiques d'image associées à la lésion à partir du segment d'image de lésion dans l'image de tomodensitométrie (10, 10') et à partir du segment d'image de lésion normalisé dans l'image de tomographie par émission de positons (20'), et
- la classification (205) de la lésion en fonction de sa gravité sur la base des valeurs de caractéristiques d'image extraites.

**11.** Procédé implémenté par ordinateur (300) de sélection d'un ensemble de caractéristiques d'image et d'une architecture d'apprentissage automatique destinés à être utilisés par un système (100) selon la revendication 1 ou dans un procédé (200) selon la revendication 10 pour la classification de lésions, comprenant :

- la fourniture (301) d'un ensemble d'images d'entraînement bimodales, chaque image d'entraînement comprenant une image de tomodensitométrie (10, 10') et une image de tomographie par émission de positons (20') recalées l'une par rapport à l'autre, dans chacune desquelles un segment d'image de lésion respectif correspondant à une lésion respective dans une région tissulaire d'intérêt a été identifié, dans lequel, pour chacune des lésions dans les images d'entraînement, une classe de gravité attribuée est fournie,
- la sélection (302) d'un ensemble candidat de caractéristiques d'image à partir d'une collection prédéfinie de caractéristiques d'image possibles,
- la génération (303) d'un ensemble de données d'entraînement par l'extraction, à partir de chacun des segments d'image de lésion identifiés dans les images d'entraînement, de valeurs correspondantes de chacune parmi l'ensemble candidat de caractéristiques d'image et leur attribution à la classe de gravité respective attribuée à la lésion dans l'image d'entraînement respective,
- la sélection (304) d'une architecture d'apprentissage automatique candidate adaptée pour recevoir des valeurs de caractéristiques d'image en tant qu'entrée et déterminer une classe de gravité de lésion associée en tant que sortie,
- l'entraînement (305) de l'architecture d'apprentissage automatique candidate sur l'ensemble de données d'entraînement,
- la fourniture (306) d'un ensemble d'images de test bimodales, chaque image de test comprenant une image de tomodensitométrie (10, 10') et une image de tomographie par émission de positons (20'), dans chacune desquelles un segment d'image de lésion respectif correspondant à une lésion respective dans une région tissulaire d'intérêt a été identifié, dans lequel, pour chacune des lésions dans les images de test, une classe de gravité attribuée est fournie,
- la génération (307) d'un ensemble de données de test par l'extraction, à partir de chacun des segments d'image de lésion identifiés dans les images de test, de valeurs correspondantes de chacune parmi l'ensemble candidat de caractéristiques d'image et leur attribution à la classe de gravité respective attribuée à la lésion dans l'image de test respective,
- la détermination (308) d'une performance de l'architecture d'apprentissage automatique candidate sur l'ensemble de données de test sur la base d'une relation entre les classes de gravité attribuées fournies pour les lésions dans les images de test et les classes de gravité de lésion correspondantes déterminées par l'architecture d'apprentissage automatique candidate,
- la répétition (309) des étapes susmentionnées avec un nombre croissant de caractéristiques d'image dans l'ensemble candidat de caractéristiques d'image, dans lequel, pour chaque répétition, l'ensemble candidat de caractéristiques d'image est agrandi par une caractéristique d'image supplémentaire de la collection prédéfinie de caractéristiques d'image possibles, la caractéristique d'image supplémentaire étant celle qui entraîne la plus forte augmentation de performance, jusqu'à ce qu'un critère d'abandon soit rempli, et
- la répétition (310) des étapes susmentionnées avec au moins une architecture d'apprentissage automatique candidate, dans lequel l'ensemble de caractéristiques d'image et l'architecture d'apprentissage automatique à utiliser pour classer des lésions sont sélectionnés parmi les candidats respectifs en fonction de la performance déterminée.

12. Procédé selon la revendication 11, dans lequel la pluralité d'architectures d'apprentissage automatique candidates correspond à des forêts aléatoires présentant différents hyperparamètres.

13. Procédé selon la revendication 12, dans lequel les hyperparamètres sont sélectionnés successivement selon un quadrillage.

14. Procédé selon l'une quelconque des revendications 11 à 13, dans lequel les images de test incluent différents sous-ensembles des images d'entraînement, réalisant ainsi une validation croisée.

15. Programme informatique de classification de lésions, le programme comprenant des moyens de code de programme pour amener un système selon l'une quelconque des revendications 1 à 9 à exécuter le procédé selon la revendication 10 lorsque le programme est lancé sur un ordinateur commandant le système.

100

image providing unit ——101

identifying unit ——102

normalizing unit ——103

image feature extracting unit ——104

classifying unit ——105

FIG. 1

3x3 conv, 64

3x3 conv, 64

*pool/2*

3x3 conv, 128

3x3 conv, 128

*pool/2*

3x3 conv, 256

3x3 conv, 256

3x3 conv, 256

*pool/2*

115

3x3 conv, 512

3x3 conv, 512

3x3 conv, 512

*pool/2*

3x3 conv, 512

3x3 conv, 512

3x3 conv, 512

FIG. 2

200

provide images — 201

identify segments — 202

normalize PET lesion image segment — 203

extract features — 204

classify lesion — 205

# FIG. 3

FIG. 4

FIG. 5

FIG. 6

Cross validation data: Confusion matrix

FIG. 7A

test data: Confusion matrix

FIG. 7B

**FIG. 8**

FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20200245960 A1

**Non-patent literature cited in the description**

- **M. BELLVER et al.** Detection-aided liver lesion segmentation using deep learning. *Conference of Neural Information Processing*, 2017, 1-4
- **K.-K. MANINIS et al.** Deep Retinal Image Understanding. *Medical Image Computing and Computer-Assisted Intervention*, 2016, vol. 9901, 140-148

- **K. SIMONYAN** ; **A. ZISSERMAN**. Very Deep Convolutional Networks for Large-Scale Image Recognition. *International Conference on Learning Representations*, 2015